# EUROPÄISCHE PATENTANMELDUNG

(11) **EP 1 675 450 A2**
(43) Veröffentlichungstag der Anmeldung: **28.06.2006**
(21) Anmeldenummer: 05026922.4
(22) Anmeldetag: 09.12.2005
(51) Int. Cl.: H05K 5/02, A61B 5/00

(54) **Elektrisches Gerät des persönlichen Bedarfs**

(30) Priorität: 23.12.2004 DE 102004062066
(71) Anmelder: Braun GmbH, 61476 Kronberg (DE)
(72) Erfinder: Freund, Dirk, Dr., 65779 Kelkheim (DE); Hollinger, Stefan, Dr., 61476 Kronberg (DE); Rönneberg, Gerrit, Dr., 64289 Darmstadt (DE); Schnak, Fred, 61476 Kronberg (DE); Simeth, Martin, 61462 Königstein (DE); Zellermayer, Siegfried, 60437 Frankfurt (DE)

(57) **Zusammenfassung**

Die vorliegende Erfindung betrifft ein elektrisches Gerät des persönlichen Bedarfs, insbesondere Blutdruckmeßgerät, mit mindestens einem ersten elektrischen Verbraucher, mit zumindest einem ersten und einem zweiten elektrischen Anschlußkabel, einer Energiequelle mit zwei elektrischen Versorgungskabeln, mit einer Leiterplatte, die einen Steckplatz aufweist und auf der eine Steuereinheit zum Ansteuern des mindestens einen ersten elektrischen Verbrauchers über den Steckplatz der Leiterplatte vorgesehen ist, wobei an den Enden der Kabel eine oder mehrere Steckverbinder vorgesehen sind, der/die mit dem Steckplatz verbindbar ist/sind und wobei beide elektrischen Versorgungskabel in den Steckverbinder münden und mit diesen elektrisch verbunden sind. Erfindungsgemäß ist das erste elektrische Kabel mit dem Steckverbinder und das zweite elektrische Kabel außerhalb der Leiterplatte mit der Energiequelle elektrisch verbunden.

## Beschreibung

Die vorliegende Erfindung betrifft eine elektrisches Gerät des persönlichen Bedarfs nach dem Oberbegriff des Anspruchs 1.

Elektrische Kleingeräte des persönlichen Bedarfs weisen in der Regel elektrische Komponenten bzw. Verbraucher wie Motoren, Ventile, Pumpen, Lichtquellen usw. auf, die elektronisch, insbesondere über eine Modulation des Energieflusses, anzusteuern sind. Dazu werden diese elektrischen Verbraucher mit einer Steuerelektronik, die auf einer elektrischen Leiterplatte angeordnet ist, elektrisch verbunden. Daneben benötigen sowohl die elektrischen Verbraucher als auch die Steuerelektronik eine Energiequelle, wie z.B. Batterien oder einen Netzanschluß, um diese mit elektrischer Energie zu versorgen. Zur Verbindung der elektrischen Verbraucher und der Energiequelle mit der Leiterplatte sind in der,Regel elektrische Kabel und ein oder mehrere Steckverbinder zwischen den Kabeln und einem Steckplatz an der Leiterplatte vorgesehen.

Zwar wird angestrebt, die elektrischen Verbraucher auf die Leiterplatte zu integrieren, um zusätzliche elektrische Verbindungen zu vermeiden, häufig stehen dem aber konstruktive oder funktionale Randbedingungen entgegen.

In vielen Geräten werden daher Kabel, Metallkontakte oder andere Verbindungstechniken eingesetzt, um die elektrische Verbindung zwischen Leiterplatte und den elektrischen Verbrauchern herzustellen. Die Verbindung zur Leiterplatte wird häufig als Steckkontakt ausgeführt, um die Endmontage des Gerätes zu vereinfachen und einen Austausch von Komponenten oder der Leiterplatte und deren separate Qualitätsprüfung zu ermöglichen.

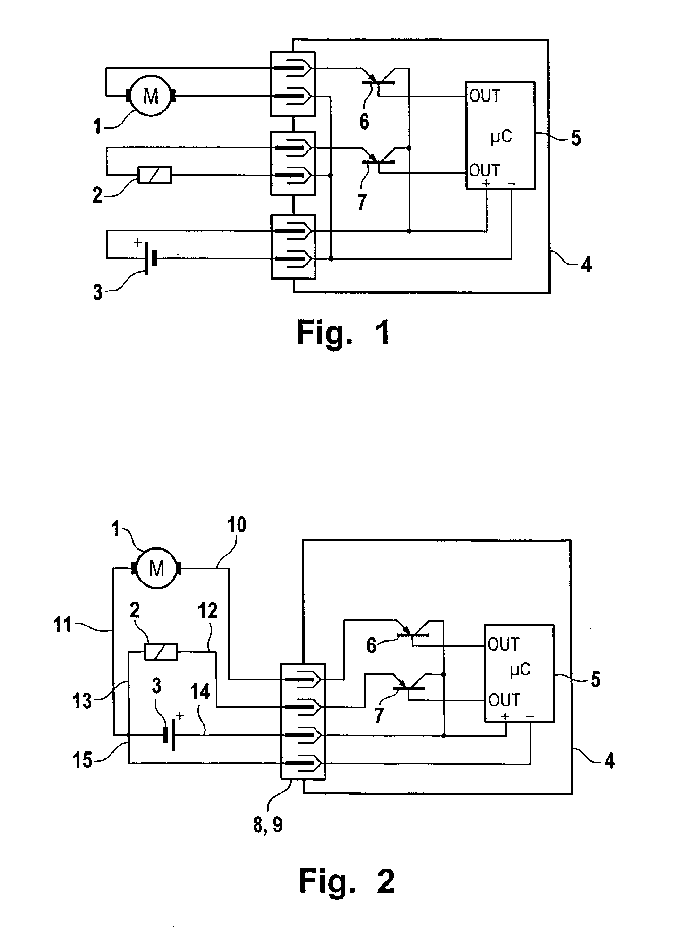

Hierzu werden alle elektrischen Leitungen der elektrischen Verbraucher und der Energiequelle zur Leiterplatte geführt und dort verschaltet (siehe Figur 1 zu diesem Stand der Technik).

In nachteiliger Weise erhöht die Anzahl der Steckverbindungsplätze oder/und die Anzahl der Pins auf einem Steckverbinder den Platzbedarf auf der Leiterplatte und im Gerät und steht somit einer kompakten und auch kostengünstigen Herstellweise entgegen. Der Stand der Technik gemäß Figur 1 weist ferner den Nachteil auf, daß die Rückströme aus den elektrischen Verbrauchern über die gemeinsame Masseleitung von der Leiterplatte zurück zur Energiequelle fließen. Die elektrischen Verbraucher weisen typischerweise eine deutlich höhere Stromaufnahme auf als die Steuerungselektronik auf der Leiterplatte. Durch den Stromfluß auf der gemeinsamen Masseleitung entsteht ein Spannungsabfall, der den Massepegel auf der Leiterplatte anhebt. Besonders störend wirkt sich dies aus, wenn die Komponenten gepulst angesteuert werden, da dann auch das Massesignal auf der Leiterplatte mit einem pulsierenden Störpegel beaufschlagt wird. Dies kann insbesondere bei Meßgeräten zu Meßungenauigkeiten führen.

Es ist daher Aufgabe der vorliegenden Erfindung, ein elektrisches Gerät der eingangs genannten Art bereit zu stellen, das besonders kompakt aufgebaut ist und das insbesondere die Signalqualität auf der Leiterplatte nicht oder weniger beeinträchtigt.

Diese Aufgabe wird durch ein elektrisches Gerät mit den Merkmalen des Anspruchs 1 gelöst.

Durch die erfindungsgemäße Lösung wird je elektrischen Verbraucher ein Steckplatz bzw. Pin am Steckverbinder eingespart, so daß hierfür weniger Platzbedarf anzusetzen ist. Dieser Vorteil wird umso signifikanter, je mehr elektrische Verbraucher vorzusehen sind. Bei einem Blutdruckmeßgerät mit typischerweise zwei elektrischen Verbrauchern, die durch eine Modulation des Energieflusses anzusteuern sind, ist dieser Vorteil bereits für ein Massenprodukt deutlich erkennbar. Weiterhin wird die Signalqualität auf der Leiterplatte deutlich verbessert, da die elektrischen Rückströme der elektrischen Verbraucher über separate Kabelleitungen unmittelbar zur Energiequelle geführt werden, so daß die Spannungsabfälle auf diesen Kabelleitungen keinen Einfluß auf den Massepegel der Leiterplatte haben.

Eine Verbindung jeweils eines der elektrischen Kabelleitungen mit der Energiequelle außerhalb der Leiterplatte kann bedeuten, daß dieses eine Kabel unmittelbar an einer Lötfahne des Metallkontakts z.B. für eine Batterie angelötet ist, so daß von diesen Metallkontakten zwei Kabel abgehen, ein erstes zum elektrischen Verbraucher und ein zweites zum Steckverbinder. Eine Verbindung außerhalb der Leiterplatte kann auch realisiert werden durch eine Verlötung oder ähnliche elektrische Verbindung und Befestigung der beiden Kabel miteinander. Denkbar ist auch, beide Kabelenden, also die des elektrischen Verbrauchers und die der Energiequelle, an einen Pin des Steckverbinders zusammenzuführen, ohne daß die Vorteile der Erfindung dadurch beeinträchtigt werden und ohne daß dadurch eine Verbindung direkt auf der Leiterplatte, wie gemäß dem Stand der Technik vorgesehen ist.

Weitere vorteilhafte Merkmale und Ausführungsformen der Erfindung gehen aus den Unteransprüchen hervor.

Bei einer Anwendung der Erfindung auf ein Blutdruckmeßgerät gemäß den Merkmalen der Ansprüche 5 und 6 wird die Signalqualität auf der Leiterplatte insbesondere dann besonders verbessert, wenn einer der elektrischen Verbraucher als Induktivität in einem Schaltwandler oder einem ähnlichen Luftablaßventil ausgebildet ist und eines dessen elektrischer Verbindungskabel unmittelbar mit dem Pluspol der Energiequelle elektrisch verbunden ist.

Weitere Ziele, Vorteile, Merkmale und Anwendungsmöglichkeiten der vorliegenden Erfindung ergeben sich aus der nachfolgenden Beschreibung von zwei Ausführungsbeispielen anhand der Zeichnungen. Dabei bilden alle beschriebenen und/oder bildlich dargestellten Merkmale für sich oder in beliebiger sinnvoller Kombination den Gegenstand der vorliegenden Erfindung, auch unabhängig von ihrer Zusammenfassung in den Ansprüche oder deren Rückbeziehung.

### Es zeigen:

Figur 1 eine schematische Schaltungsanordnung für ein Blutdruckmeßgerät gemäß dem Stand der Technik,

Figur 2 eine schematische Schaltungsanordnung gemäß einer ersten Ausführungsform der Erfindung und Figur 3 eine schematische Schaltungsanordnung gemäß einer zweiten Ausführungsform der Erfindung.

Gleiche Komponenten in den Figuren sind mit gleichen Bezugsziffern versehen, so daß diese nicht für alle Figuren wiederholt beschrieben werden.

Alle Figuren 1 bis 3 zeigen schematisch elektrische Schaltungsanordnungen für ein Blutdruckmeßgerät. Das Blutdruckmeßgerät weist einen ersten elektrischen Verbraucher 1 auf, der als Pumpenmotor ausgebildet ist. Durch den Pumpenmotor wird Luft angesaugt und in eine Manschette des Blutdruckmeßgerätes (nicht dargestellt) gepumpt. Die Manschette wird üblicherweise am Handgelenk des Unterarms angelegt und alle in den Figuren dargestellten Komponenten sind in einem Gehäuse angeordnet, das fest mit der Manschette mechanisch befestigt ist und in Fluidverbindung mit dieser steht.

Als zweiter elektrischer Verbraucher 2 ist ein Luftablaßventil vorgesehen, mit dem gesteuert die Luft aus der Manschette abgelassen werden kann. Beide elektrischen Verbraucher sind Komponenten, die eine oszillometrische Blutdruckmessung am Handgelenk des Menschen erlauben.

Das Blutdruckmeßgerät weist ferner eine Energiequelle 3 auf, die in Form von Batterien bereitgestellt wird.

Das Blutdruckmeßgerät weist neben allen üblichen in Handgelenksblutdruckmeßgeräten nach der oszillometrischen Meßmethode vorgesehenen Teile noch eine Leiterplatte 4 auf, auf der eine Steuereinheit 5 vorgesehen ist. Die Steuereinheit 5 weist ihrerseits zumindest zwei elektrische Verbindungen auf der Leiterplatte zur Energieversorgung und zumindest zwei Ausgänge auf, die mit den elektrischen Verbrauchern 1, 2 in elektrischer Verbindung stehen, so daß die elektrischen Verbraucher in ihrer Arbeitsweise durch den Mikroprozessor ansteuerbar sind. Hierzu sind als exemplarische elektrische Lösung die beiden Ausgänge der Steuereinheit für die jeweiligen beiden elektrischen Verbraucher je mit einer Transistorschaltung verbunden, so daß das Steuersignal des Mikroprozessors die Basis der jeweiligen Transistoren 6, 7 ansteuert. Je eine weitere elektrische Verbindung der Transistoren ist mit einer der elektrischen Zuleitungen 10, 12 der jeweiligen elektrischen Verbraucher 1, 2 verbunden und die jeweilige andere elektrische Anschlußmöglichkeit der Transistoren 6, 7 steht mit dem Pluspol der Energiequelle in elektrischer Verbindung. Bei einem positiven Steuersignal auf die Basis des jeweiligen Transistors wird somit der jeweilige elektrische Verbraucher mit Strom versorgt. Diese können auch in gepulster Form angesteuert werden.

Die Leiterplatte 4 weist ferner einen oder mehrere anteilig mit der Leiterplatte ausgebildete Steckplätze auf, die mit einem oder mehreren Steckverbindern verbindbar sind.

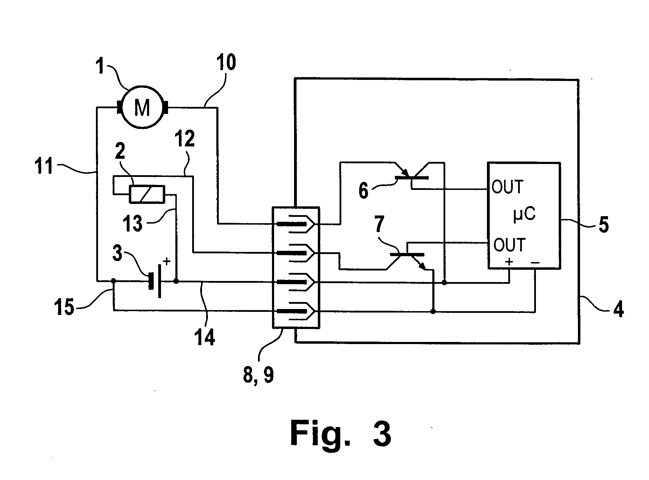

Gemäß Figur 2 und 3 sind zwei elektrische Verbindungspunkte des einzigen Steckplatzes 8 auf der Leiterplatte mit dem Plus- und dem Minuspol des Mikroprozessors 5 verbunden. Die beiden anderen elektrischen Verbindungspunkte des Steckplatzes 8 stellen eine elektrische Verbindung 10, 12 zu je einen der elektrischen Verbraucher 1, 2 her.

An den Steckplatz der Leiterplatte ist ein Steckverbinder 9 mit vier Pins bzw. vier elektrischen Verbindungspunkten oder Belegungsplätzen aufsteckbar. Der erste elektrische Verbindungspunkt des Steckverbinders ist über ein Kabel 10 mit dem Pumpenmotor 1 elektrisch verbunden. Der zweite elektrische Verbindungspunkt des Steckverbinders 9 ist mit dem Luftablaßventil 2 über ein Kabel 12 elektrisch verbunden. Der dritte elektrische Verbindungspunkt des Steckverbinders ist mit dem Pluspol der metallischen Kontaktfahne für die Batterien mittels des Kabels 14 verbunden. Der vierte elektrische Verbindungspunkt des Steckverbinders ist mit der Masse der Energiequelle über ein elektrisches Kabel 15 verbunden.

Bei der Ausführung gemäß Figur 2 ist das elektrische Kabel, das nicht mit dem Steckverbinder, wie soeben beschrieben, verbunden ist, mit dem Metallkontakt der Batterien für die Masse elektrisch verbunden. Die elektrische Verbindung wird dadurch hergestellt, daß die Kabelenenden am Metallkontakt angelötet werden. Somit laufen am Massepol des Batteriekontaktes drei Kabel zusammen, die dort angelötet sind.

Bei der Ausführungsform nach Figur 3 ist unverändert wie in Figur 2 auch das elektrische Kabel 11, das nicht wie oben beschrieben mit dem Steckverbinder 9 elektrisch verbunden ist und in diesen mündet mit dem Massepol des Metallkontaktes für die Batterieaufnahme elektrisch durch Anlöten verbunden. Das weitere elektrische Kabel 13 des Luftablaßventils 2, das nicht mit dem Steckverbinder, wie oben beschrieben, elektrisch verbunden ist, wird am Metallkontakt des Pluspols der Batterieaufnahme angelötet, so daß eine elektrische Verbindung mit dem Pluspol der Energiequelle hergestellt ist.

## Patentansprüche

1. Elektrisches Gerät des persönlichen Bedarfs, insbesondere Blutdruckmeßgerät, mit mindestens einem ersten elektrischen Verbraucher (1), mit zumindest einem ersten und einem zweiten elektrischen Anschlußkabel (10, 11), einer Energiequelle (3) mit zwei elektrischen Versorgungskabeln (14, 15), mit einer Leiterplatte (4), die einen Steckplatz (8) aufweist und auf der eine Steuereinheit (5) zum Ansteuern des mindestens einen ersten elektrischen Verbrauchers (1) über den Steckplatz (8) der Leiterplatte (4) vorgesehen ist, wobei an den Enden der Kabel (10) eine oder mehrere Steckverbinder (9) vorgesehen sind, der/die mit dem Steckplatz (8) verbindbar ist/sind und wobei beide elektrischen Versorgungskabel (14, 15) in den Steckverbinder (9) münden und mit diesen elektrisch verbunden sind, **dadurch gekennzeichnet, daß** das erste elektrische Kabel (10) mit dem Steckverbinder und das zweite elektrische Kabel (11) außerhalb der Leiterplatte (4) mit der Energiequelle (3) elektrisch verbunden sind.

2. Gerät nach Anspruch 1, **dadurch gekennzeichnet, daß** ein zweiter elektrischer Verbraucher (2) mit einem dritten und vierten elektrischen Anschlußkabel (12, 13) vorgesehen ist, der ebenfalls von der Steuereinheit (5) ansteuerbar ist, wobei das dritte Kabel (12) in den Steckverbinder (9) mündet und mit diesem elektrisch verbunden ist und daß vierte Kabel (13) außerhalb der Leiterplatte (4) mit der Energiequelle (3) elektrisch verbunden ist.

3. Gerät nach Anspruch 1 oder 2, **dadurch gekennzeichnet, daß** das zweite und das vierte elektrische Kabel (11, 13) mit dem gleichen Pol der Energiequelle (3), insbesondere mit der Masse der Energiequelle elektrisch verbunden sind.

4. Gerät nach Anspruch 1 oder 2, **dadurch gekennzeichnet, daß** das zweite und das vierte elektrische Anschlußkabel (11, 13) mit je verschiedenen Polen der Energiequelle (3) elektrisch verbunden sind.

5. Gerät nach Anspruch 2, **dadurch gekennzeichnet, daß** als erster elektrischer Verbraucher (1) ein Pumpenmotor und als zweiter elektrischer Verbraucher (2) ein Luftablaßventil, insbesondere eine Induktivität in einem Schaltwandler, vorgesehen sind.

6. Gerät nach Anspruch 5, **dadurch gekennzeichnet, daß** das zweite elektrische Kabel (11) mit der Masse und das vierte elektrische Kabel (13) mit dem Pluspol der Energiequelle (3) elektrisch verbunden sind.

7. Gerät nach einem der Ansprüche 2 bis 6, **dadurch gekennzeichnet, daß** der Steckverbinder (9) vier elektrische Steckverbindungen aufweist.
